# EUROPÄISCHE PATENTANMELDUNG

(11) **EP 0 522 576 A2**
(43) Veröffentlichungstag der Anmeldung: **13.01.1993**
(21) Anmeldenummer: 92111763.6
(22) Anmeldetag: 10.07.1992
(51) Int. Cl.: C07F 9/38, C07F 9/40, C07F 9/30, A61K 31/66

(54) **Substituierte Aminoethan-1,1-bisphosphonsäuren und Aminoethan-1,1-alkylphosphinphosphonsäuren, Verfahren zu ihrer Herstellung und Verwendung derselben**

(30) Priorität: 12.07.1991 DE 4123025
(71) Anmelder: HOECHST AKTIENGESELLSCHAFT, D-65926 Frankfurt am Main (DE)
(72) Erfinder: Lang, Hans-Jochen, Dr., W-6238 Hofheim/Taunus (DE); Naumann, Christoph, Dr., W-6272 Niedernhausen (DE); Raiss, Ruth, Dr., W-6000 Frankfurt/Main (DE); Komiyama, Osamu, Nakano-ku, Tokyo (JP); Terui, Yoshiko, Kokubunji, Tokyo (JP)

(57) **Zusammenfassung**

Die Erfindung betrifft Verbindungen der Formel I, 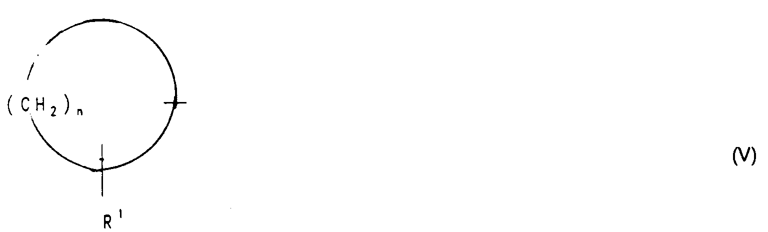
wobei R¹, R², R³, R⁴ oder R⁵ für Wasserstoffatom oder (C₁-C₅)-Alkyl stehen, n für eine ganze Zahl von 3 bis 10, m für die ganze Zahl 0 oder 1, x für (C₁-C₁₀)-Alkyl, ein- oder mehrfach substituiert, 5- oder 6-gliedriger Ring mit 1 N-Atom, gegebenenfalls einem weiteren N- oder O-Atom, Aryl mit 6 oder 10 Kohlenstoffatomen, die substituiert sein können, oder Cycloalkyl steht, Verfahren zur Herstellung der Verbindung der Formel I, Arzneimittel, sowie deren Verwendung zur Behandlung von Osteoporose und/oder degenerativen Gelenkserkrankungen.

## Beschreibung

Osteoporose ist eine häufig vorkommende Knochenerkrankung. Bei den verschiedenen Formen der Osteoporose kommt es zu einem starken Verlust an Knochengewebe, so daß schließlich die mechanische Stabilität des Knochens verloren geht. In gesunden Menschen ist die Rate mit der Osteoclasten und Osteoplasten gebildet werden so ausgebildet, daß Knochenbildung und Knochenresorption im Gleichgewicht stehen. Bei Osteoporose ist das Gleichgewicht gestört, so daß es zu einem Knochenabbau kommt.

Arthrose ist eine degenerative Gelenkserkrankung mit entzündlichen Episoden und progredienter Knorpelzerstörung, die zu Funktionsbeeinträchtigung bis hin zu völliger Versteifung führen kann. Bisher sind zwar Entzündung und Schmerz bei dieser Erkrankung therapierbar, es gibt jedoch bisher kein Arzneimittel, das erwiesenermaßen den fortschreitenden Knorpelabbau aufzuhalten bzw. zu heilen vermag. Bekannte Therapeutika der Arthrose sind beispielsweise Mischungen von sulfatierten Glucosaminoglycanen (Current Therapeutic Research, 40,6 (1986), 1034) oder nichtsteroidale Antiinflammatorika, die jedoch den Knorpelverlust nicht aufzuhalten vermögen.

Die Pathogenese der Arthrose ist noch nicht im Einzelnen aufgeklärt, es wird aber zur Zeit als gesichert angenommen, daß die Chondrozyten (Knorpelzellen) maßgeblich an dem verstärkten Matrixverlust beteiligt sind, und daß von den Hauptbestandteilen dieser Matrix vor allem die Proteoglykane (PG) als erste enzymatisch abgebaut werden.

Die Europäische Patentanmeldung EP-A-0 325 482 beschreibt (Cycloalkylamino)methanbisphosphonsäuren und deren Verwendung als Arzneimittel zur Verminderung der Knochenresorption und als Antiarthritismittel. Die Europäische Patentanmeldung EP-A-0 407 344 beschreibt topisch applizierbare pharmazeutische Präparate enthaltend Methandiphosphonsäurederivate.

In dem Bestreben wirksame Verbindungen zur gleichzeitigen Behandlung und Prophylaxe von degenerativen Gelenkserkrankungen und Osteoporose mit geringen Nebenwirkungen zu erhalten, wurde nun überraschenderweise gefunden, daß die erfindungsgemäß substituierten Aminoethan-1,1-bisphosphonsäuren oder Aminoethan-1,1-alkylphosphinphosphonsäuren die in vitro Synthese von Knorpelmatrix-Proteoglykanen erhöhen, den Knorpelabbau inhibieren, die Proteoglykansynthese im Knorpel erhöhen und die knochenresorption vermindern.

Die Erfindung betrifft daher Verbindungen der Formel I, 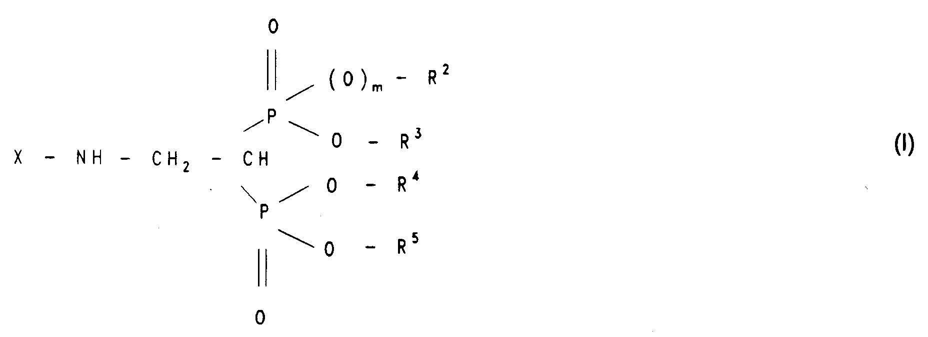
und/oder deren physiologisch verträglichen Salze, wobei
a)
   - R¹, R², R³, R⁴ oder R⁵: gleich oder verschieden sind und für
   - 1): Wasserstoffatom oder
   - 2): (C₁-C₅)-Alkyl, geradkettig oder verzweigt, stehen,
   - x: für den Rest der Formel V 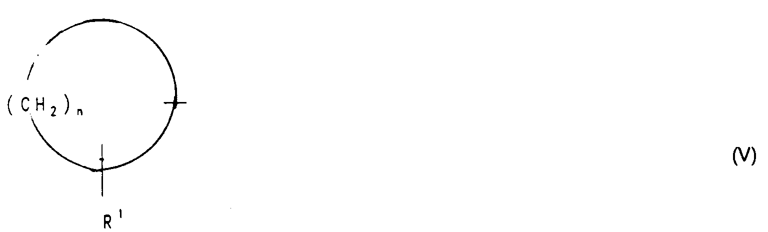
   - n: für eine ganze Zahl von 3 bis 10 und
   - m: für Null oder 1 steht, oder
b)
   - m: für die ganze Zahl 0 steht,
   - R², R³, R⁴ oder R⁵: gleich oder verschieden sind und für
   - 1): Wasserstoffatom oder
   - 2): (C₁-C₅)-Alkyl, geradkettig oder verzweigt, stehen und
   - x: für
   - 1): (C₁-C₁₀)-Alkyl, geradkettig oder verzweigt,
   - 2): (C₁-C₁₀)-Alkyl, geradkettig oder verzeigt, ein- oder mehrfach substituiert durch
   2.1. Halogen wie Chlor-, Brom-, Jod- oder Fluoratom,
   2.2. -NH₂,
   2.3. 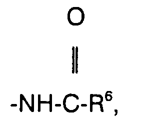 worin R⁶ für (C₁-C₆)-Alkyl steht,
   2.4. -N-(R⁷)₂, worin R⁷ unabhängig voneinander für
      2.4.1. Wasserstoffatom oder
      2.4.2. (C₁-C₃)-Alkyl steht,
   2.5. Hydroxyl,
   2.6. 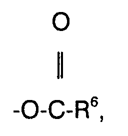
   worin R⁶ für (C₁-C₆)-Alkyl steht,
   - 3): monocyclischer 5- bis 6-gliedriger gesättigter oder ungesättigter heterocyclischer Rest mit 1 N-Atom, wobei gegebenenfalls ein C-Atom durch ein N-, S- oder O-Atom im Ring ersetzt ist und ein-oder mehrfach substituiert ist durch
   3.1. Wasserstoffatom oder
   3.2. wie unter 2.1. bis 2.6. definiert,
   - 4): Aryl, mit 6 oder 10 Kohlenstoffatomen,
   - 5): Aryl, mit 6 oder 10 Kohlenstoffatomen, ein-oder mehrfach substituiert, wie unter 2.1. bis 2.6. definiert, steht.

Unter dem Rest der Formel V werden Reste wie beispielsweise Cyclopropyl, Cyclobutyl, Cyclopentyl, Cyclohexyl, Cycloheptyl, Cyclooctyl, Cyclononyl oder Cyclodecyl verstanden, die gegebenenfalls durch eine oder mehrere Alkylreste mit 1 bis 5 Kohlenstoffatomen substituiert sein können. Für den Fall das m für die ganze Zahl O steht, ergeben sich die entsprechend substituierten Aminoethan-1,1-phosphinphosphonsäuren.

Unter monocyclischen 5- bis 6-gliedrigen gesättigten oder ungesättigten heterocyclischen Rest mit 1 N-Atom werden Verbindungen verstanden, die sich von Pyrrol, Pyrrolin, Pyrrolidin, Pyridin, Tetrahydropyridin oder Piperidin ableiten.

Enthält der obengenannte 5- bis 6-gliedrigen Rest ein weiteres N-Atom für ein C-Atom, so ergeben sich Verbindungen, die sich von Pyrazol, Imidazol, Pyrazolin, Imidazolin, Pyrazolidin, Imidazolidin, Pyridazin, Pyrimidin, Pyrazin oder Piperazin ableiten.

Enthält der obengenannte 5- bis 6-gliedrige Rest ein O-Atom für ein C-Atom, so ergeben sich Verbindungen, die sich von Oxazol, Isoxazol, Isoxazolin, Isoxazolidin oder Morpholin ableiten.

Enthält der obengenannte 5- bis 6-gliedrige Rest ein S-Atom für ein C-Atom, so ergeben sich Verbindungen, die sich von Thiazol, Isothiazol, Thiazolin, Isothiazolin oder Thiazin ableiten.

Bevorzugt sind die Verbindungen der Formel I, wobei
a)
   - m: für die ganze Zahl 1 steht,
   - R¹, R², R³, R⁴ oder R⁵: gleich oder verschieden sind und für
   - 1): Wasserstoffatom oder
   - 2): (C₁-C₅)-Alkyl, geradkettig oder verzweigt, stehen,
   - x: für den Rest der Formel V steht, 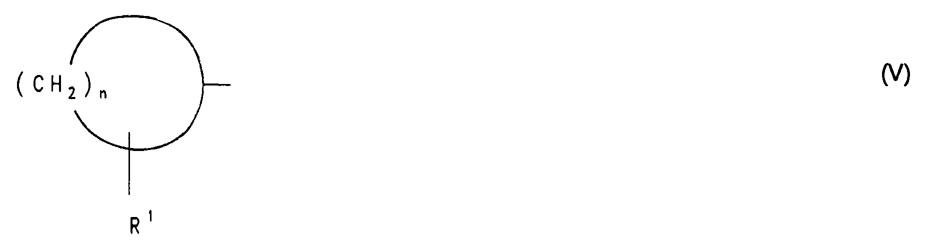 wobei n für eine ganze Zahl 8, 9 oder 10 steht,
b)
   - m: für Null steht,
   - R¹, R², R³, R⁴ oder R⁵: gleich oder verschieden sind und für
   - 1): Wasserstoffatom oder
   - 2): (C₁-C₅)-Alkyl, geradkettig oder verzweigt, stehen,
   - x: für den Rest der Formel V steht, wobei
   - n: für eine ganze Zahl von 3 bis 10 steht oder
c)
   - m: für Null steht,
   - R², R³, R⁴ oder R⁵: gleich oder verschieden sind und für
   - 1): Wasserstoffatom oder
   - 2): (C₁-C₅)-Alkyl, geradkettig oder verzweigt, stehen und
   - x: für
   - 1): (C₁-C₁₀)-Alkyl, geradkettig oder verzweigt,
   - 2): (C₁-C₁₀)-Alkyl, geradkettig oder verzweigt, ein- oder mehrfach substituiert durch
   2.1. Halogen wie Chlor-, Brom-, Jod- oder Fluoratom,
   2.2. -NH₂,
   2.3. 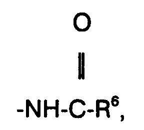 worin R⁶ für (C₁-C₆)-Alkyl steht,
   2.4. -N-(R⁷)₂,
      worin R⁷ unabhängig voneinander für
      2.4.1. Wasserstoffatom oder
      2.4.2. (C₁-C₃)-Alkyl steht,
   2.5. Hydroxyl,
   2.6. 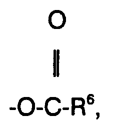 worin R⁶ für (C₁-C₆)-Alkyl steht,
   - 3): monocyclischer 5- bis 6-gliedriger gesättigter oder ungesättigter heterocyclischer Rest mit 1 N-Atom, wobei gegebenenfalls ein C-Atom durch ein N-, S- oder O-Atom im Ring ersetzt ist und ein- oder mehrfach substituiert ist durch
   3.1. Wasserstoffatom oder
   3.2. wie unter 2.1. bis 2.6. definiert,
   - 4): Aryl, mit 6 oder 10 Kohlenstoffatomen,
   - 5): Aryl, mit 6 oder 10 Kohlenstoffatomen, ein- oder mehrfach substituiert, wie unter 2.1. bis 2.6. definiert, steht.

Besonders bevorzugt sind Verbindungen der Formel 1, wobei
a)
   - m: für die ganze Zahl 1 steht,
   - R¹, R², R³, R⁴ und R⁵: für Wasserstoffatom stehen und
   - n: für eine ganze Zahl 8, 9 oder 10 steht,
b)
   - m: für Null steht,
   - R¹, R², R³, R⁴ und R⁵: für Wasserstoffatom stehen und
   - n: für eine ganze Zahl von 5 bis 7 steht oder
c)
   - m: für Null steht,
   - R², R³, R⁴ und R⁵: für Wasserstoffatom stehen und
   - x: für
   - 1): (C₁-C₅)-Alkyl, geradkettig oder verzweigt,
   - 2): einen Rest aus der Gruppe
   2.1. Piperidinyl,
   2.2. Pyrolidinyl,
   2.3. Piperazinyl,
   2.4. Pyridinyl oder
   2.5. Morpholinyl,
   - 3): Phenyl oder
   Naphthyl steht.

Insbesondere bevorzugt sind die Verbindungen aus folgender Gruppe:
Tetraethyl-2-(cyclohexylamino)-ethan-1,1-bisphosphonat,
Tetraethyl-2-(cycloheptylamino)-ethan-1,1-bisphosphonat,
Tetraethyl-2-(cyclooctylamino)-ethan-1,1-bisphosphonat oder
2-(Cyclooctylamino)-ethan-1,1-bisphosphonsäure.

Geeignete physiologisch verträgliche Salze von den Verbindungen der Formel I sind beispielsweise Alkali-, Erdalkali- oder Ammoniumsalze, sowie solche von physiologisch verträglichen, organischen Ammonium- oder Triethylaminbasen.

Zur Herstellung der erfindungsgemäßen Verbindungen geht man wie folgt vor:
a) Ethylenbisphosphonsäurederivat der Formel II, 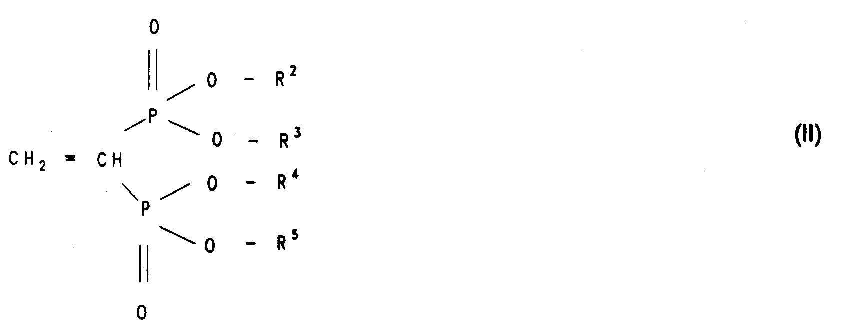 wobei R², R³, R⁴ oder R⁵ für Wasserstoffatom oder Alkyl mit 1 bis 5 Kohlenstoffatomen stehen, wird mit Cycloalkylamin der Formel III, 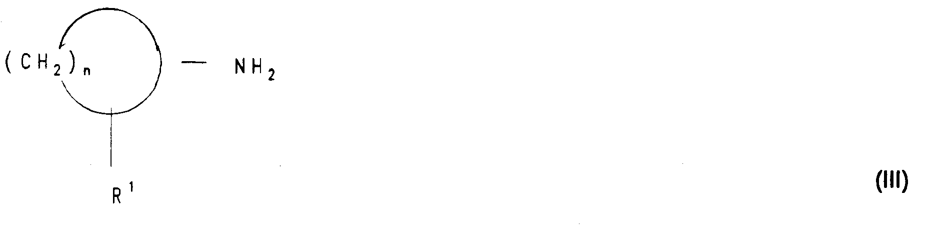 wobei n für eine ganze Zahl von 3 bis 10 und R¹ für Wasserstoffatom oder Alkyl mit 1 bis 5 Kohlenstoffatomen steht, zu der Verbindung der Formel I umgesetzt, oder
b) Ethylenphosphinphosphonsäurederivat der Formel IV, 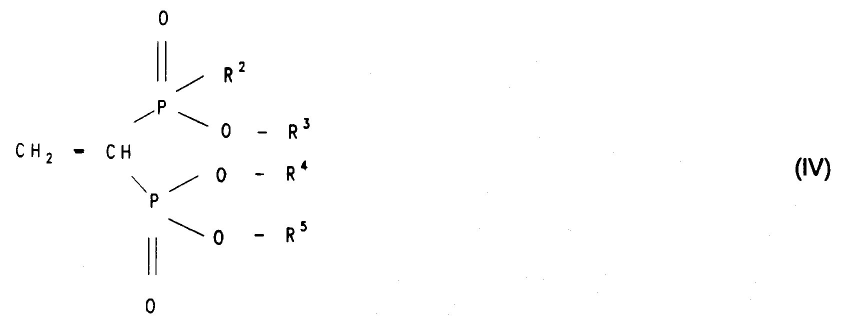 wobei R², R³, R⁴ oder R⁵ die unter a) genannte Bedeutung haben, wird mit Cycloalkylamin der Formel III oder der Verbindung der Formel x-NH₂, wobei x die obengenannte Bedeutung hat, zu der Verbindung der Formel I umgesetzt, oder
c) die nach den Verfahren a) oder b) erhaltenen Verbindungen werden zu der Verbindung der Formel I hydrolysiert, oder
d) die Verbindungen der Formel I wird mit einer Base in die entsprechenden Salze überführt.

Bei der Verfahrensvariante a) geht man am besten so vor, daß man die Verbindung der Formel III in äquimolaren Mengen oder in einem bis zu 3-fachen Überschuß, gegebenenfalls in einem inerten Lösungsmittel wie beispielsweise Dimethylsulfoxid (DMSO), Dimethylformamid (DMF), Toluol, Tetrahydrofuran (THF), Dioxan oder Diethylether, mit einer Verbindung der Formel II bis zur Beendigung der Reaktion umsetzt. Die Reaktionstemperaturen liegen dabei zwischen 25 °C und 100 °C, vorzugsweise bei Verwendung eines Lösungsmittels zwischen 25 °C und dem Siedepunkt des Lösungsmittels, insbesondere bei 70 °C. Die Reaktionszeiten liegen zwischen 6 und 24 Stunden, bevorzugt zwischen 12 und 20 Stunden. Die Beendigung der Reaktion kann beispielsweise mittels Dünnschichtchromatographie (Laufmittel Methanol/Chloroform 1: 40) bestimmt werden. Die Umsetzung kann auch wie bei H. O. House beschrieben (Modern Synthetic Reaction 2nd Ed. W. A. Benjamin Inc. S. 595 - 623) durchgeführt werden.

Die Ausgangsverbindungen für die Verfahrensvariante a), die Verbindungen der Formel II und III sind, sofern nicht käuflich, auf einfache Weise nach literaturbekannten Verfahren herstellbar (EP-A-0 298 558).

Bei der Verfahrensvariante b) geht man wie unter a) beschrieben vor.

Bei der Verfahrensvariante c) gebt man am besten so vor, daß man die nach den Verfahrensvarianten a) oder b) erhaltenen Verbindungen in konzentrieter Salzsäure unter Rückfluß hydrolysiert oder mit Trimethylsilyhalogenid (EP-A-0 298 553) eine Teilhydrolyse durchführt. Die Reaktionstemperatur liegt bei Verwendung von Salzsäure zwischen 80 °C und 120 °C, insbesondere zwischen 90 °C und 100 °C. Die Reaktionszeiten liegen zwischen 1 Stunde und 6 Stunden, bevorzugt zwischen 2 Stunden und 4 Stunden. Bei Verwendung von Trimethylsilyhalogenid liegen die Reaktionstemperaturen zwischen 0 °C und 70 °C, bevorzugt zwischen 20 °C und 40 °C. Die Reaktionszeiten liegen zwischen 1 Stunde und 4 Stunden.

Bei der Verfahrensvariante d) geht man am besten so vor, daß man die nach den Verfahrensvarianten a), b) oder c) erhaltenen Verbindungen der Formel I mit einer Base, wie Natriumhydroxid, Kaliumhydroxid, Ammonium oder organische Amine, in die entsprechenden Salze überführt.

Gegenstand der Erfindung sind auch Arzneimittel, die mindestens eine Verbindung der Formel I und/oder mindestens eines von deren physiologisch verträglichen Salze enthalten, neben pharmazeutisch geeigneten und physiologisch verträglichen Hilfs- und Trägerstoffen, Verdünnungsmitteln und/oder anderen Wirkstoffen.

Die vorliegende Erfindung betrifft ferner die Verwendung der erfindungsgemäßen Verbindungen der Formel I und/oder deren physiologisch verträglichen Salze zur Prophylaxe und Behandlung von degenerativer Gelenkserkrankungen, dazu gehören z. B. Arthrosen, Arthrosen mit entzündlichen Episoden und progredienter Knorpelzerstörung, die von der Funktionsbeeinträchtigung bis zur völligen Versteifung der Gelenke reichen können, Erkrankungen des rheumatischen Formenkreises mit Knorpelabbau, chronischer Polyarthritis, Knorpelschwund nach Gelenktrauma, beispielsweise nach Meniskus- oder Patellaverletzungen oder Bänderrissen, oder bei Knorpelschwund nach längerer Stillegung von Gelenken.

Ein weiterer Gegenstand der Erfindung ist die Verwendung der Verbindungen der Formel I und/oder deren physiologisch verträglichen Salze zur Prophylaxe und Behandlung von Osteoporose.

Die erfindungsgemäßen Arzneimittel können intravenös, intramuskulär, intraperitoneal, subcutan, intraartikulär, periartikulär, rektal oder oral verabreicht werden.

Die Herstellung der erfindungsgemäßen Arzneimittel zur Behandlung degenerativer Gelenkserkrankungen erfolgt, indem mindestens eine Verbindung der Formel I und/oder eines von deren physiologisch verträglichen Salze gegebenenfalls mit weiteren Hilfs- und/oder Trägerstoffen in eine geeignete Darreichungsform gebracht wird. Die Hilfs- und Trägerstoffe stammen aus der Gruppe der Trägermittel, Konservierungsstoffe und anderer üblicher Hilfsstoffe.

Zum Beispiel können für orale Darreichungsformen Hilfsstoffe wie Stärken, z. B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate vewendet werden. Weiterhin ist es vorteilhaft, den oralen Darreichungsformen Hilfsstoffe zuzusetzen, die die Verträglichkeit der Medikamente verbessern, wie z. B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente eines Kombinationspräparates, ein Retardierungsmittel zuzusetzen, gegebenenfalls in Form von permeablen Membranen, wie z. B. solche auf Cellulose- oder Polystrolharzbasis oder Ionenaustauscher.

Die anzuwendende Dosierung der erfindungsgemäßen Arzneimittel ist abhängig von verschiedenen Faktoren wie Darreichungsform des Medikamentes und Zustand, Gewicht und Schwere der Erkrankung des Patienten. Eine Tagesdosis von ca. 5000 mg der erfindungsgemäßen Arzneimittel sollte jedoch nur kurzzeitig überschritten werden. Bevorzugt sind ca. 10 bis 2500 mg als Tagesdosis für einen Menschen von ca. 70 kg Körpergewicht. Die Verabreichung der Tagesdosis der erfindungsgemäßen Arzneimittel kann in Form einer einzelnen Verabreichung oder in mehreren kleinen Dosen erfolgen. Bevorzugt ist die Verabreichung in 3 bis 8 Dosen pro Tag.

Die Wirksamkeit der erfindungsgemäßen Verbindungen der Formel I wird in vitro in folgenden Versuchen nachgewiesen:

### 1. Wirksamkeit in der Synthese-Stimulierung von Knorpel-Matrix, Prüfung in der Chondrozyten-Kultur

Zellen: Den Fußgelenken frisch geschlachteter Rinder werden der hyaline Knorpel entnommen, mit Pronase (Boehringer Mannheim) und Collagenase (Sigma) die native Matrix enzymatisch abgebaut, und die Chondrozyten in 1 %iger "low melting Agarose" in 24er Multiwell-Schalen in einer Zelldichte von 4 x 10⁶ pro well ausplattiert.

Medium: Komplettes Medium enthält HAM's F12 (Biochrom KG, Berlin) und 10 % foetales Kälberserum (Boehringer Mannheim), die Testsubstanz wird in Medium gelöst, in einer Konzentration von 10⁻⁵M zugegeben und bei jedem Mediumwechsel erneut zugefügt.

Versuchs-Durchführung: Die Behandlung erfolgt vom dritten bis zehnten Tag der Primärkultur, am 9. Tag wird 20µC/ml Na₂ ³⁵SO₄ (7,4 x 10⁵ Bq) für 24 h in dem Medium zugefügt. Die dissoziative Extraktion der Proteoglykane aus der Agarose-Schicht wird mit 8M Guanidinium-Hydrochlorid und beigefügten Proteinase-Inhibitoren (Sigma) unter Schütteln bei 4 °C über 24 h durchgeführt. Der Überstand wird nach Zentrifugation über eine PD 10 ®Sephadex G 25 Säule in freies und inkorporiertes Sulfat getrennt, dessen Aktivität nach Aliquotierung im β-Szintillationscounter gemessen wird.

Auswertung: Der Parameter für die Matrixproduktion der Chondrozyten ist die Menge synthetisierter Proteoglykane; gemessen als Sulfatinkorporation in cpm. Aus vier wells pro Gruppe wird der Mittelwert errechnet und als Prozentwert bezogen auf die unbehandelte Kontrollgruppe = 100 % angegeben.

Ergebnisse: Die Ergebnisse sind in Tab. 1 aufgeführt.

**Tabelle 1**

| Stimulierung der Proteoglykan-Synthese in der Agarose-Zellkultur | |
|---|---|
| Präparat Beispiel | % ³⁵SO₄-Inkorporation bezogen auf unbehandelte Kontrolle = 100 % |
| C | 107 |
| D | 110 |

### 2. Wirksamkeit in der chondrozytären Chondrolyse, Prüfung in der Knorpel-Explant-Kultur

### Methodenbeschreibung:

Knorpelgewebe: Aus den Fußgelenken frisch geschlachteter Rinder werden genormte Scheiben hyalinen Knorpels ausgestanzt und in 24er Multiwell-Schalen mit ca. 200 mg pro well kultiviert.

Medium: Dem kompletten Medium wie in Test 1 wird zusätzlich ab Behandlungsbeginn 10 u/ml humanes rec. Interleukin-1 alpha zugesetzt, das wie die Testsubstanz bei jedem Mediumwechsel erneut zugefügt wird. Die Testsubstanz wird in einer Konzentration von 10⁻⁵M eingesetzt.

Versuchs-Durchführung: Die Behandlung erfolgt vom 3. bis 10. Tag der Explantkuitur, am 9. Tag wird 20µC/ml Na₂³⁵SO₄ für 24 h dem Medium zugefügt. Die dissoziative und anschließend assoziative Extraktion der Proteoglykane aus den Knorpelexplanten wird mit 8M Guanidinium-Hydrochlorid und beigefügten Proteinase-Inhibitoren unter mehrfachem Tieffrieren und Auftauen durchgeführt. Der Überstand wird nach Zentrifugation über eine PD 10 Sephadex G 25 Säule in freies und inkorporiertes Sulfat getrennt, dessen Aktivität nach Aliquotierung im β-Szintillationscounter gemessen wird. Aus sechs wells pro Gruppe wird der Mittelwert errechnet und als Prozentwert bezogen auf die unbehandelte Kontrollgruppe = 100 % angegeben.

Auswertung: IL-1 führt zu einer Synthesehemmung sowie zu einer Degradationssteigerung der Proteoglykane, was sich im geringeren Gehalt an markierten Matrixmolekülen im Knorpelexplantat widerspiegelt. Die Prozentwerte werden berechnet wie in Test 1. Ergebnisse: Die Ergebnisse sind in Tab. 2 aufgeführt.

**Tabelle 2**

| Einfluß auf IL-1-induzierte Chondrolyse in der Knorpel-Explantkultur | |
|---|---|
| Präparat Beispiel | % ³⁵SO₄-Inkorporation bezogen auf unbehandelte Kontrolle = 100 % |
| IL-1 Kontrolle | 76 |
| IL-1 + Verbindung | |
| nach Beispiel | |
| C | 91 |
| D | 85 |

### Herstellungsbeispiel A

Man erhitzt 7,5 g Tetraethylethylidenbis(phosphonat) und 2,5 g Cyclohexylamin in 50 ml THF 17 Stunden lang zum sieden (etwa 70 °C). Nach Einengen der Reaktionslösung unter vermindertem Druck wird der Rückstand im Silikagelsäulenchromatographen (Methanol/Chloroform = 1/40) gereinigt. Ausbeute: 7,5 g Tetraethyl-2-(cyclohexylamino)-ethan-1,1-bis(phosphonat) als gelbe, ölige Substanz.

| | | | | |
|---|---|---|---|---|
| Analyse: | ber. | C = 48.1 | H = 8.8 | N = 3.5 |
| | gef. | C = 48.0 | H = 8.7 | N = 3.4 |

Massenspektrum (m/z): 399
¹H-NMR-Spektroskopie:
(CDCl₃, TMS) 1.12 (t, 12H, OCH₂CH₃); 1.51 - 2.53(10H, aliphat); 2.55 - 3.18 (1H, CH); 3.35 - 4.12(3H); 4.16 - 4.54(8H, OCH₂CH₃).

### Beispiel B

Tetraethyl-2-(cycloheptylamino)ethan-1,1-bis(phosphonat) wird wie in Beispiel A aus Cycloheptylamin und Ethylenbisphosphonsäuretetraethylester hergestellt.

| | | | | |
|---|---|---|---|---|
| Analyse: | ber. | C = 49.4 | H = 8.5 | N = 3.4 |
| | gef. | C = 49.5 | H = 8.7 | N = 3.4 |

gelbe, ölige Substanz
Massenspektrum (m/z): 413
¹H-NMR-Spektroskopie:
(CDCl₃, TMS) 1.33 (t, 12H, OCH₂CH₃); 1.20 - 2.03 (13H, aliphat); 2.30 - 2.88 (1H, CH); 2.95 - 3.33 (2H, -CH₂-); 4.03 - 4.33 (8H, OCH₂CH₃)

### Beispiel C

6 g Tetraethyl 2-(cyclohexylamino)ethan-1,1-bis(phosphonat) aus Beispiel A werden in 40 ml konzentrierter Salzsäure gelöst und 4 Stunden zum Sieden (100 °C) erhitzt. Nach dem Abkühlen wird die Reaktionslösung unter vermindertem Druck eingeengt und die Salzsäure entfernt. Das hierbei erhaltene harzige Produkt wird mit 30 ml einer Mischung aus Aceton/Wasser=10:1 aufgeschlämmt, wobei 2-(Cyclohexylamino)ethan-1,1-bis(phosphonat) als ein farbloses Pulver erhalten wird.
Ausbeute: 3,8 g.

| | | | | |
|---|---|---|---|---|
| Analyse: | ber. | C = 33.4 | H = 6.6 | N = 4.9 |
| | gef. | C = 33.3 | H = 6.6 | N = 4.9 |

Schmelzpunkt: 226 - 231°C (Zersetzung)
¹H-NMR-Spektroskopie:
(D₂O/Na₂CO₃) 1.0 - 2.0 (ₘ,11H, aliphat); 2.4 (mc, 1H, CH); 2.9 (mc, 2H, - CH₂-).
³¹P-NMR-Spektroskopie:
(NaOD) 20.24 ppm.

### Beispiel D

2-(Cycloheptylamino)ethan-1,1-bisphosphonsäure wird wie im Beispiel C aus der Verbindung die im Beispiel B erhalten wurde hergestellt.
Ausbeute: 3,6 g.

| | | | | |
|---|---|---|---|---|
| Analyse: | ber. | C = 35.8 | H = 6.7 | N = 4.7 |
| | gef. | C = 35.6 | H = 6.9 | N = 4.8 |

Schmelzpunkt: 225 °C (Zersetzung)
¹H-NMR-Spektroskopie:
(D₂O/Na₂CO₃) 1.3 - 1.9 (ₘ,13H, aliphat); 2.7 (mc, 1H, CH); 2.8 (mc, 2H, - CH₂-). ³¹P-NMR-Spektroskopie:
(NaOD) 19.84 ppm.

### Beispiel E

9,45 g (31,5 mMol) Tetraethylidenbis(phosphonat) wird in 40 ml Ethanol gelöst und auf 5°C gekühlt. Hierzu tropft man bei 5°C 4 g (31,5 mMol) Cyclooctylamin gelöst in 10 ml Ethanol zu. Nach der Zugabe wird 24 h bei Raumtemperatur gerührt. Das Lösungsmittel wird im Vakuum entfernt. Dabei wird das Produkt bereits in reiner Form erhalten.
Ausbeute: 13,1 g Tetraethyl-2-(cyclooctylamino)-ethan-1,1-bis(phosphonat) als gelbe, ölige Substanz.

| | | | | |
|---|---|---|---|---|
| Analyse: | ber. | C = 50.6 | H = 9.2 | N = 3.3 |
| | gef. | C = 50.3 | H = 8.7 | H = 3.7 |

Massenspektrum (m/z): 427
¹H-NMR-Spektroskopie:
(CDCl₃, TMS) 1.35 (t, 12H, OCH₂CH₃); 1.43 - 1.85 (14H, aliphat); 2.60 - 2.69(1H, cyclo-CH); 2.51 - 2.70 (1H, -CH-); 3.07 - 3.21(2H, -CH₂); 4.13 - 4.25(8H, OCH₂CH₃).
³¹P-NMR-Spektroskopie
(CDCl₃) 23.24 ppm.

### Beispiel F

2-(Cyclooctylamino)-ethan-1,1-bisphosphonsäure wird wie im Beispiel C aus der Verbindung die im Beispiel E erhalten wurde hergestellt.
Ausbeute: 3,3 g

| | | | | |
|---|---|---|---|---|
| Analyse: | ber. | C = 38.1 | H = 7.4 | N = 4.4 |
| | gef. | C = 38.2 | H = 7.3 | N = 4.4 |

Schmelzpunkt: 241°C
¹H-NMR-Spektroskopie:
(D₂O/Na₂CO₃) 1.43 - 1.85 (14H, aliphat); 1.74 - 1.93(1H, -CH-); 2.76 - 2.87(1H, cyclo-CH); 2.88 - 3.07 (1H, -CH-).
³¹P-NMR-Spektroskopie:
(NaOD) 19.60 ppm.

## Patentansprüche

1. Verbindung der Formel I, 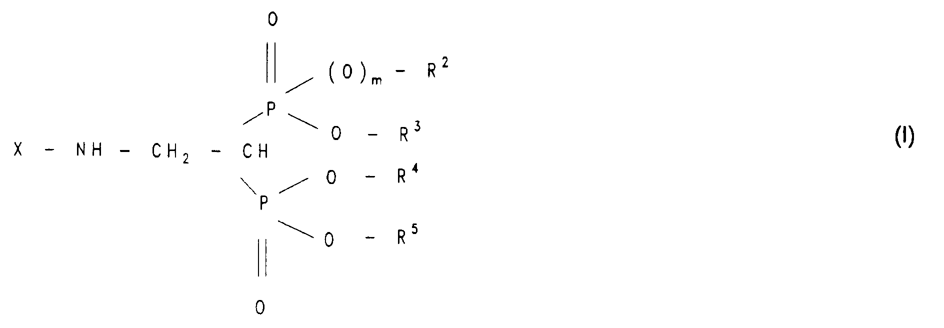 und/oder deren physiologisch verträglichen Salze, wobei
a)
R¹, R², R³, R⁴ oder R⁵ gleich oder verschieden sind und für
1) Wasserstoffatom oder
2) (C₁-C₅)-Alkyl, geradkettig oder verzweigt, stehen,
x für den Rest der Formel V 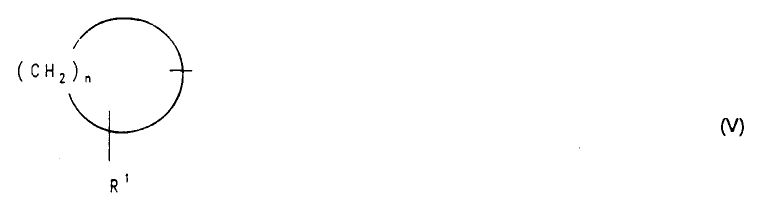
n für eine ganze Zahl von 3 bis 10 und
m für Null oder 1 steht oder
b)
m für die ganze Zahl 0 Steht,
R², R³, R⁴ oder R⁵ gleich oder verschieden sind und für
1) Wasserstoffatom oder
2) (C₁-C₅)-Alkyl, geradkettig oder verzweigt, stehen und
x für
1) (C₁-C₁₀)-Alkyl, geradkettig oder verzweigt,
2) (C₁-C₁₀)-Alkyl, geradkettig oder verzweigt, ein- oder mehrfach substituiert durch
2.1. Halogen wie Chlor-, Brom-, Jod- oder Fluoratom,
2.2. -NH₂,
2.3. 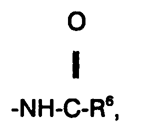 worin R⁶ für (C₁-C₆)-Alkyl steht,
2.4. -N-(R⁷)₂, worin R⁷ unabhängig voneinander für
2.4.1. Wasserstoffatom oder
2.4.2. (C₁-C₃)-Alkyl steht,
2.5. Hydroxyl,
2.6. 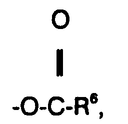 worin R⁶ für (C₁-C₆)-Alkyl steht,
3) monocyclischer 5- bis 6-gliedriger gesättigter oder ungesättigter heterocyclischer Rest mit 1 N-Atom, wobei gegebenenfalls ein C-Atom durch ein N-, S- oder O-Atom im Ring ersetzt ist und ein- oder mehrfach substituiert ist durch 3.1. Wasserstoffatom oder 3.2. wie unter 2.1. bis 2.6. definiert,
4) Aryl, mit 6 oder 10 Kohlenstoffatomen,
5) Aryl, mit 6 oder 10 Kohlenstoffatomen, ein- oder mehrfach substituiert, wie unter 2.1. bis 2.6. definiert, steht.

2. Verbindung der Formel I nach Anspruch 1, wobei
a)
m für die ganze Zahl 1 steht,
R¹, R², R³, R⁴ oder R⁵ gleich oder verschieden sind und für
1) Wasserstoffatom oder
2) (C₁-C₅)-Alkyl, geradkettig oder verzweigt, stehen,
x für den Rest der Formel V steht, 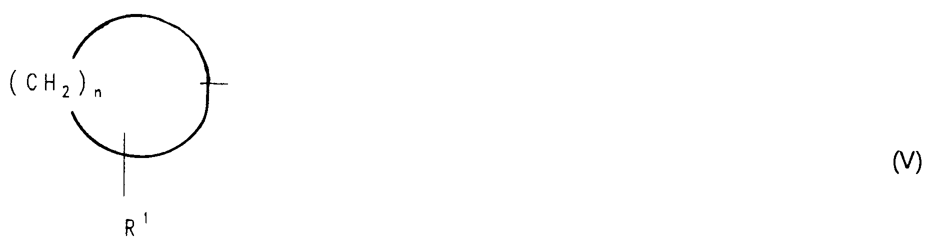
wobei n für eine ganze Zahl 8,9 oder 10 steht,
b)
m für Null steht,
R¹, R², R³, R⁴ oder R⁵ gleich oder verschieden sind und für
1) Wasserstoffatom oder
2) (C₁-C₅)-Alkyl, geradkettig oder verzweigt, stehen,
x für den Rest der Formel V steht, wobei
n für eine ganze Zahl von 3 bis 10, steht oder
c)
m für Null steht,
R², R³, R⁴ oder R⁵ gleich oder verschieden sind für
1) Wasserstoffatom oder
2) (C₁-C₅)-Alkyl, geradkettig oder verzweigt, stehen und
x für
1) (C₁-C₁₀)-Alkyl, geradkettig oder verzweigt,
2) (C₁-C₁₀)-Alkyl, geradkettig oder verzweigt, ein-oder mehrfach substituiert durch
2.1. Halogen wie Chlor-, Brom-, Jod- oder Fluoratom,
2.2. -NH₂,
2.3. 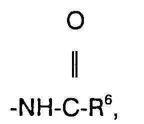 worin R⁶ für (C₁-C₆)-Alkyl steht,
2.4. -N-(R⁷)₂, worin R⁷ unabhängig voneinander für
2.4.1. Wasserstoffatom oder
2.4.2. (C₁-C₃)-Alkyl steht,
2.5. Hydroxyl,
2.6. 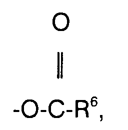 worin R⁶ für (C₁-C₆)-Alkyl steht,
3) monocyclischer 5- bis 6-gliedriger gesättigter oder ungesättigter heterocyclischer Rest mit 1 N-Atom, wobei gegebenenfalls ein C-Atom durch ein N-, S- oder O-Atom im Ring ersetzt ist und ein- oder mehrfach substituiert ist durch
3.1. Wasserstoffatom oder
3.2. wie unter 2.1. bis 2.6. definiert,
4) Aryl, mit 6 oder 10 Kohlenstoffatomen,
5) Aryl, mit 6 oder 10 Kohlenstoffatomen, ein- oder mehrfach substituiert, wie unter 2.1. bis 2.6. definiert, steht.

3. Verbindungen der Formel I nach Anspruch 1 oder 2, wobei
a)
m für die ganze Zahl 1 steht,
R¹, R², R³, R⁴ und R⁵ für Wasserstoffatom stehen und
n für eine ganze Zahl 8, 9 oder 10 steht
b)
m für Null steht,
R¹, R², R³, R⁴ und R⁵ für Wasserstoffatom stehen und
n für eine ganze Zahl von 5 bis 7 steht oder
c)
m für Null steht,
R², R³, R⁴ und R⁵ für Wasserstoffatom stehen und
x für
1) (C₁-C₅)-Alkyl, geradkettig oder verzweigt,
2) einen Rest aus der Gruppe
2.1. Piperidinyl,
2.2. Pyrolidinyl,
2.3. Piperazinyl,
2.4. Pyridinyl oder
2.5. Morpholinyl,
3) Phenyl oder
Naphthyl steht.

4. Tetraethyl-2-(cyclohexylamino)-ethan-1,1-bisphosphonat
Tetraethyl-2-(cycloheptylamino)-ethan-1,1-bisphosphonat
Tetraethyl-2-(cyclooctylamino)-ethan-1,1-bisphosphonat oder
2-(Cyclooctylamino)-ethan-1,1-bisphosphonsäure.

5. Verfahren zur Herstellung der Verbindung der Formel I nach Anspruch 1, dadurch gekennzeichnet, daß man
a) Ethylenbisphosphonsäurederivate der Formel II, 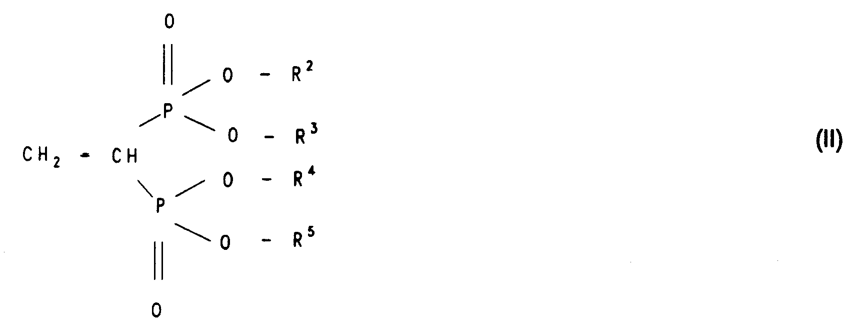 wobei R², R³, R⁴ oder R⁵ für Wasserstoffatom oder Alkyl mit 1 bis 5 Kohlenstoffatomen stehen, wird mit Cycloalkylamin der Formel III, 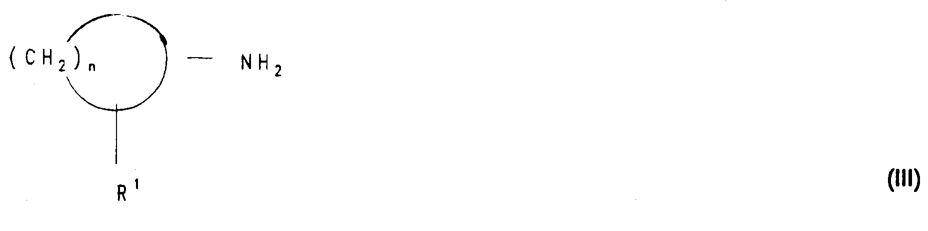 wobei n für eine ganze Zahl von 3 bis 10 und R¹ für Wasserstoffatom oder Alkyl mit 1 bis 5 Kohlenstoffatomen steht, zu der Verbindung der Formel I umgesetzt, oder
b) Ethylenphosphinphosphonsäurederivat der Formel IV, 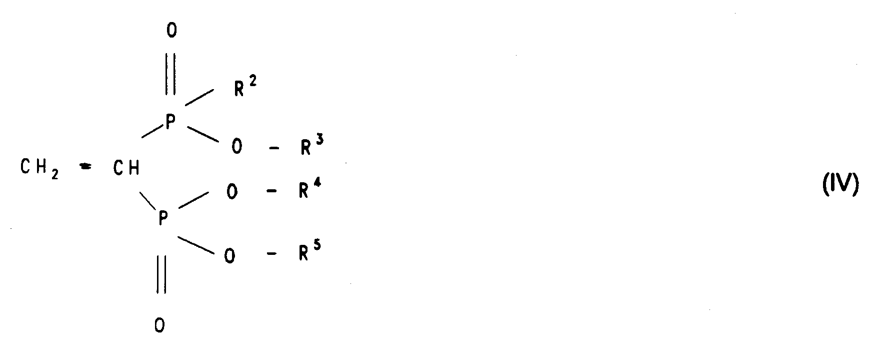 wobei R², R³, R⁴ oder R⁵ die unter a) genannte Bedeutung haben, wird mit Cycloalkylamin der Formel III oder der Verbindung der Formel x-NH₂, wobei x die in Anspruch 1 genannte Bedeutung hat, zu der Verbindung der Formel I umgesetzt, oder
c) die nach den Verfahren a) oder b) erhaltenen Verbindungen werden zu der Verbindung der Formel I hydrolysiert, oder
d) die Verbindung der Formel I wird mit einer Base in die entsprechenden Salze überführt.

6. Arzneimittel, enthaltend eine wirksame Menge mindestens einer Verbindung der Formel I nach einem oder mehreren der Ansprüche 1 bis 4 oder wie erhalten nach Anspruch 5 und/oder mindestens ein physiologisches verträgliches Salz einer solchen Verbindung neben physiologisch annehmbaren Hilfs- und Trägerstoffen, gegebenenfalls weiteren Zusatzstoffen und/oder anderen Wirkstoffen.

7. Verfahren zur Herstellung eines Arzneimittels nach Anspruch 6, dadurch gekennzeichnet, daß man mindestens eine Verbindung der Formel I und/oder mindestens ein physiologisch verträgliches Salz der Verbindung der Formel I und/oder eine nach dem Verfahren nach Anspruch 5 erhaltene Verbindung mit physiologisch annehmbaren Hilfs- und Trägerstoffen, gegebenenfalls weiteren Zusatzstoffen und/oder anderen Wirkstoffen in eine geeignete Darreichungsform bringt.

8. Verwendung der Verbindung der Formel I nach einem oder mehreren der Ansprüche 1 bis 4 und/oder wie erhalten nach dem Verfahren nach Anspruch 5 zur Herstellung eines Arzneimittels zur Prophylaxe und Behandlung von degenerativen Gelenkserkrankungen.

9. Verwendung nach Anspruch 8 zur Herstellung eines Arzneimittels zur Stimulierung der Proteoglykan-Synthese und/oder zur Verminderung des Knorpelabbaus.

10. Verwendung der Verbindung der Formel I nach einem oder mehreren der Ansprüche 1 bis 4 und/oder erhalten nach dem Verfahren nach Anspruch 5 zur Herstellung eines Arzneimittels zur Prophylaxe und Behandlung von Osteoporose.
